(19) **Europäisches Patentamt / European Patent Office / Office européen des brevets**

(11) **EP 3 537 983 B1**

(12) **EUROPEAN PATENT SPECIFICATION**

(45) Date of publication and mention of the grant of the patent:
**15.07.2020 Bulletin 2020/29**

(21) Application number: **17808343.2**

(22) Date of filing: **09.11.2017**

(51) Int Cl.:
**A61B 8/06** *(2006.01)*     **A61B 8/08** *(2006.01)*

(86) International application number:
**PCT/EP2017/078690**

(87) International publication number:
**WO 2018/087198 (17.05.2018 Gazette 2018/20)**

(54) **SYSTEM AND METHOD FOR CHARACTERIZING LIVER PERFUSION OF CONTRAST AGENT FLOW**

ULTRASCHALLDIAGNOSESYSTEM UND VERFAHREN ZUR KONTRASTVERSTÄRKTEN LEBERDIAGNOSE

SYSTÈME DE DIAGNOSTIC ULTRASONORE ET PROCÉDÉ DE DIAGNOSTIC DU FOIE À CONTRASTE RENFORCÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.11.2016 PCT/CN2016/105682**
**20.03.2017 EP 17161867**

(43) Date of publication of application:
**18.09.2019 Bulletin 2019/38**

(73) Proprietor: **Koninklijke Philips N.V.**
**5656 AG Eindhoven (NL)**

(72) Inventors:
- **GU, Xiaolin**
  **5656 AE Eindhoven (NL)**
- **DENG, Yinhui**
  **5656 AE Eindhoven (NL)**
- **LI, Xiaomin**
  **5656 AE Eindhoven (NL)**
- **SHAMDASANI, Vijay Thakur**
  **5656 AE Eindhoven (NL)**

(74) Representative: **Mumbrú Forn, José**
**Société Civile SPID**
**33, rue de Verdun**
**92150 Suresnes (FR)**

(56) References cited:
**WO-A1-2009/093211     WO-A1-2011/041244**

- LUECK G J ET AL: "Hepatic Perfusion Imaging Using Factor Analysis of Contrast Enhanced Ultrasound", IEEE TRANSACTIONS ON MEDICAL IMAGING, IEEE SERVICE CENTER, PISCATAWAY, NJ, US, vol. 27, no. 10, 1 October 2008 (2008-10-01), pages 1449-1457, XP011226135, ISSN: 0278-0062, DOI: 10.1109/TMI.2008.922695
- THOMAS P. GAUTHIER ET AL: "Reproducibility of Quantitative Assessment of Altered Hepatic Hemodynamics With Dynamic Contrast-Enhanced Ultrasound", JOURNAL OF ULTRASOUND IN MEDICINE., vol. 31, no. 9, 18 January 2012 (2012-01-18), pages 1413-1420, XP055408157, United States ISSN: 0278-4297, DOI: 10.7863/jum.2012.31.9.1413
- DONG LIU ET AL: "Hepatic Perfusion Parameters of Contrast-Enhanced Ultrasonography Correlate With the Severity of Chronic Liver Disease", ULTRASOUND IN MEDICINE AND BIOLOGY., vol. 40, no. 11, 1 November 2014 (2014-11-01), pages 2556-2563, XP055408158, US ISSN: 0301-5629, DOI: 10.1016/j.ultrasmedbio.2014.05.011

EP 3 537 983 B1

## Description

### FIELD OF THE INVENTION

This invention relates to systems for characterizing liver perfusion of contrast agent flow and, in particular, to systems which processess contrast-enhanced ultrasound image data. Contrast-enhanced ultrasound imaging is known to be able to assist the clinicians to identify and characterize lesions such as liver tumors.

Hepatitis B and hepatitis C patients have been found to be at an increased risk of developing primary liver cancer, hepatocellular carcinoma (HCC). Due to the discovery that hepatitis C was being contracted by patients through blood transfusions in the early 1980's, there remain a significant number of hepatitis C patients who need to be examined regularly for the onset of HCC, as the lesions are best treated in their early stages. The usual progression of the disease is from hepatitis to liver cirrhosis to HCC. An easy-to-use monitoring technique for liver disease progression would have widespread application in assisting in the early detection of this serious disease.

There are a number of lesions in addition to HCC which can develop in the liver. The paper "Ultrasound Contrast Imaging Research" by M. Averkiou et al., published in Ultrasound Quarterly, vol. 19, no. 1 at pp 27 et seq. (2003) discusses four primary liver lesions: hepatocellular carcinoma (HCC), metastasis from a primary tumor at some other location, hemangioma, and focal nodal hyperplasia. The former two are malignant and the latter two are benign. A technique for identifying lesions in the liver should be able to detect all four of these lesions so that more detailed diagnosis can identify and distinguish between all lesions found in the liver. The paper "Hepatic Perfusion Imaging Using Factor Analysis of Contrast Enhanced Ultrasound" by G. J. Lueck et al., published in IEEE TRANSACTIONS ON MEDICAL IMAGING, vol. 27, no. 10 at pp 1449 et seq. (2008) discloses the features of the preamble of claim 1. This paper presents a method of using factor analysis for visual separation of two different phases of contrast-enhancement from the independent feeding vessels, namely the hepatic artery and the portal vein. WO2011/041244 A1 discloses a method for assessing a liver comprising steps of identifying the location of the main hepatic artery (MHA) and the location of the main portal vein (MPV) in at least one contrast-enhanced ultrasound image of the liver, obtaining time-intensity information corresonding to perfusion of contrast agent in the MHA and MOV, and determining a biomarker index value.

### SUMMARY OF THE INVENTION

Since liver lesions, like other cancers, are most effectively treated when detected early, high-risk patients should be monitored frequently for signs of these diseases. But in their early stages liver lesions are often difficult to detect through conventional diagnostic imaging due to their small size. Thus, clinicians often conduct their diagnoses to look for other signs that a lesion is developing. One of these signs is changes in the blood flow to the liver. The liver has a unique blood supply network. A primary source of fresh blood to the liver is the arterial inflow from the hepatic artery. But the liver has a secondary blood supply, the portal vein in the abdomen. Being both arterial and venous, these sources of supply function differently. The pulsatile flow of blood from the hepatic artery occurs shortly after systole, like other arterial vessels. The inflow of blood from the portal vein occurs later in the heart cycle. The relative timing and amount of blood flow from these two sources has been related to the onset of lesions in the liver. Thus, it is desirable to be able to analyze the characteristics of blood flow in the liver from these two sources.

However, the flow of blood in the liver parenchyma is a combination of blood flows from these two sources. It is not possible to discern separately the amount of blood arriving in regions of interest in the liver from these separate sources. It is desirable to be able to separate the flow of blood in different regions of interest in the liver as it relates to the hepatic arterial and portal venous flows.

The present invention provides a system as defined in claim 1, a method as defined in claim 9, and a computer program product as defined in claim 15. Preferred embodiments are defined in the dependent claims.

In accordance with the present invention a system and method are described for identifying the sources of blood flow in regions of interest in the liver as being arterial or venous. The present inventors have noted that the combined blood flow in the liver is linearly related to inflows from both the hepatic artery and the portal vein. The time-intensity curve (TIC) of blood flow in the hepatic artery, and optionally additionally the TIC of blood flow in the portal vein are used as references for these separate sources of blood supply. A time-intensity curve of the perfusion of blood in a region of interest (ROI) in the liver parenchyma. A scaled replica of the hepatic artery TIC is subtracted from the TIC of the ROI to separate the TIC into two curves, one relating to hepatic arterial inflow and the other relating to portal vein inflow.

Moreover, the present inventors have noted that he initial portion of the TIC of the ROI, when inflow is only from the hepatic artery, is mostly related to the hepatic artery time-intensity curve. Hence, in accordance with some embodiments of the present invention, the whole time-intensity curve of contrast agent flow in the hepatic artery is scaled in amplitude such that the initial poriton of the time-intensity curve of contrast agent flow in the hepatic artery matches the time-intensity in the ROI. The initial portion can be a region or a part of the region when inflow is substantially only from the hepatic artery, which can be, for example, demarcated by time interval from the arrival of contrast from the hepatic artery to the later time of arrival of contrast from a portal vein of the liver. The arrival of contrast from the hepatic artery in the ROI can be estimiated as equal to the arrival of contrast in the ROI. The arrival time of contrast from the portal vein in the ROI can be presumbly estimated as being later than the arrival of the contrast in the ROI by a predetermined time interval. Additionally or alternatively, the arrival time of contrast from the portal vein in the ROI can be estimated on basis of a time-intensity curve of contrast agent flow in the portal vein.

In accordance with some further embodiments, the time-intensity curve of contrast agent flow in the hepatic artery can be further scaled in time. For example, the scaling in time can be implemented as a time shift.

The time-invensity curves (TICs) of contrast agent flow in the hepatic artery, the portal vein and in the ROI can be provided in various ways. In some example, there is a source for contrast-enhanced ultrasound (CEUS) image data of the liver, and the TICs are produced from the contrast-enhanced ultrasound image data. The source of such CEUS image data can be a local or a remote storage medium. The source of such CEUS image data can be an ultrasound probe. In some other example, there is a source for direclty providing the TICs, which can be a local or remote storage medium, or can be a time-intensity curve processor.

In accordance with some further embodiments, an output unit is provided for outputting one or more of the time-intensity curve data representative of arterial flow in the ROI and the time-intensity curve data representative of venous flow in the ROI. The time-intensity curve data can be one or more chacteristics of the time-intensity curves of any one or both sources of blood flow in the liver. The output unit can comprise a user interface, such as a display. Additionally or alternatively, the output unit can comprise a data communication unit for sending out the time-intensity curve data via any kind of communication link. In this way, a separate user interface can receive the time-intensity curve data and then present the data to the users.

The time-intensity curve data can be presented in various ways. In some embodiments, one or more characteric parameters of the TICs of one or more ROIs can be presented in texture, graphical, or audio format. In some embodiments, a parameter map representing a parameter for each imaging point in an imaging region can be produced and displayed. For an example, a parametric map of the time-intensity curve data representative of arterial flow in the liver or the time-intensity curve data representative of venous flow in the liver can be produced. Additionally, the parameteric map can be overlaid on a B-mode image of the liver.

In accordance with some further embodiments, a user control can be provided to select an ROI in a liver image. Additionally, the user control is further configured to apply the spatial location of an ROI in a liver image to the time-intensity curve processor.

In accordance with a first aspect of the invention, there is provided a system for characterizing liver perfusion of contrast agent flow on basis of ultrasound data acquired from a liver in the presence of contrast agent flow. The system comprises: a first source of time-intensity curves of contrast agent flow in a hepatic artery, and in at least one region of interest, i.e. ROI, in liver parenchyma of the liver, the time-intensity curves being produced from the ultrasound data; and the system is characterized by a time-intensity curve scalar and subtractor, which is configured to produce, for an ROI of the at least one ROI, time-intensity curve data representative of arterial flow in the ROI by scaling the time-intensity curve of contrast agent flow in the hepatic artery to the time-intensity curve of contrast agent flow in the ROI, and to produce time-intensity curve data representative of venous flow in the ROI by subtracting the time-intensity curve data representative of arterial flow in the ROI from the time-intensity curve of contrast agent flow in the ROI.

In some embodiments, the first source comprises: a second source of ultrasound liver image data acquried in the presence of contrast agent flow; a nonlinear signal separator, coupled to receive the ultrasound liver image data, and configured to produce harmonic echo signal data from contrast agent flow; and a time-intensity curve processor, responsive to the harmonic echo signal data, which is configured to produce the time-intensity curves of contrast agent flow in the hepatic artery, and in the at least one ROI.

In accordance with a second aspect of the invention, there is provided a method of characterizing liver perfusion of contrast agent flow on basis of ultrasound liver image data acquired from a liver in the presence of contrast agent flow. The method comprises steps of acquiring a time-intensity curve of contrast agent flow in a hepatic artery location, and a time-intensity curve of contrast agent flow in each of at least one region of interest, i.e. ROI, in the liver parenchyma of the liver, wherein the time-intensitity curves are produced from the ultrasound liver image data. The method is characterized by steps of, for a ROI of the at least one ROI, producing time-intensity curve data representative of arterial flow in the ROI by scaling the time-intensity curve of contrast agent flow in the hepatic artery location to match the time-intensity curve of contrast agent flow in the ROI, and producing time-intensity curve data representative of venous flow in the ROI by subtracting the time-intensity curve data representative of arterial flow in the ROI from the time-intensity curve of contrast agent flow in the ROI.

In accordance with a third aspect of the invention, there is provided a computer program product comprising instructions which, when the computer program product is executed by a computer, cause the computer to carry out the method.

BRIEF DESCRIPTION OF THE DRAWINGS

In the drawings:

FIGURE 1 is an illustration of the liver, showing the entry of the hepatic artery and portal vein into the bottom of the organ.

FIGURE 2 illustrates contrast and B mode images of a liver showing the location of the hepatic artery entering the liver.

FIGURE 3 illustrates contrast and B mode images of a liver showing the location of the portal vein entering the liver.

FIGURE 4 illustrates time-intensity curves of contrast flow in the hepatic artery and in the portal vein.

FIGURE 5 illustrates a simplified time-intensity curve of contrast flow in the hepatic artery.

FIGURE 6 illustrates a simplified time-intensity curve of contrast flow in the portal vein.

FIGURE 7 illustrates a typical time-intensity curve of perfusion in a region of interest in the liver.

FIGURE 8 illustrates in block diagram form an ultrasonic diagnostic imaging system constructed in accordance with the present invention.

FIGURE 9 is a flow chart of a method for conducting liver diagnosis in accordance with the present invention.

## DETAILED DESCRIPTION OF THE EMBODIMENTS

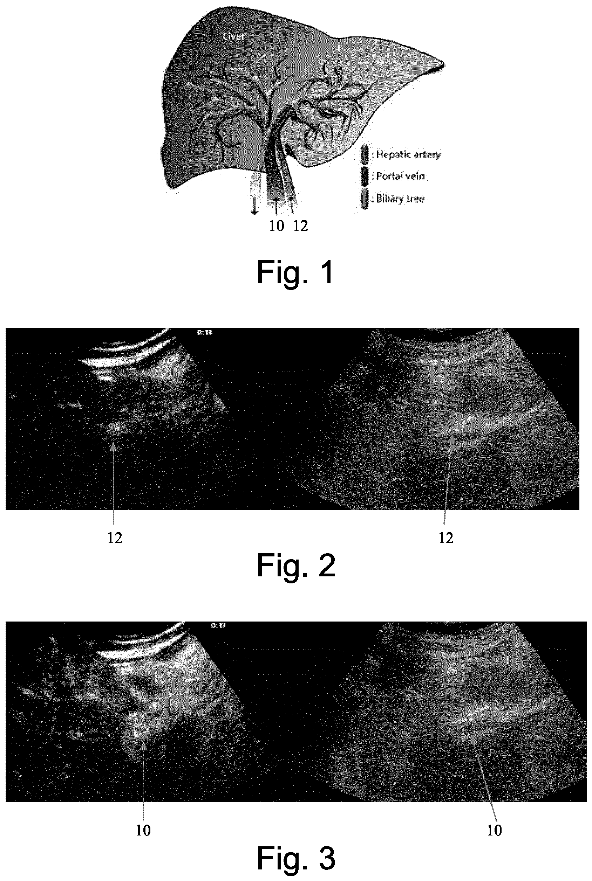

FIGURE 1 is an illustration of the liver and its flow network. The liver contains vessel trees for three sources of flow: the biliary tree for the flow of liver bile, the portal vein 10 which provides a nourishing flow of blood from the abdomen, and the hepatic artery 12 which is a source of arterial blood flow. The capillary structures of these networks are intertwined throughout the parenchyma, causing liver tissue to be perfused with blood from both arterial and venous sources. The hepatic artery 12 and portal vein 10 can be accessed separately where they enter the organ at the bottom of the liver as shown in the illustration.

FIGURE 2 shows two ultrasound images, a contrast image on the left and a B mode image on the right, illustrating the location of the hepatic artery 12 and its blood flow. The two images were acquired at the same time with the vascular system perfused with a microbubble contrast agent. The contrast image on the left was processed by enhancing the harmonic (nonlinear) signal return from microbubbles while suppressing fundamental signal frequencies from tissue, whereas the B mode image on the right was produced from fundamental frequency signals with harmonic frequencies suppressed.

The hepatic artery 12 is outlined in both images. The hepatic artery 12 is easier to locate in the left contrast image due to its cyclical brightening with each heartbeat as contrast perfused blood is pumped through the vessel. Showing both the contrast and tissue images at the same time assists the sonographer in stabilizing the probe during image acquisition. The right tissue image, in which contrast flow is suppressed, should be relatively static and helps the sonographer maintain the probe constantly aligned with the same scan plane, while the pulsatile flow in the more dynamic contrast image assists in spotting the hepatic artery from its cyclical blood flow.

FIGURE 3 is another pair of matching contrast and tissue images, this time with a tracing of the portal vein 10 added to the images. Consistent with the illustration of FIGURE 1, it is seen that the hepatic artery 12 and portal vein 10 can be accessed for scanning adjacent to each other below the liver.

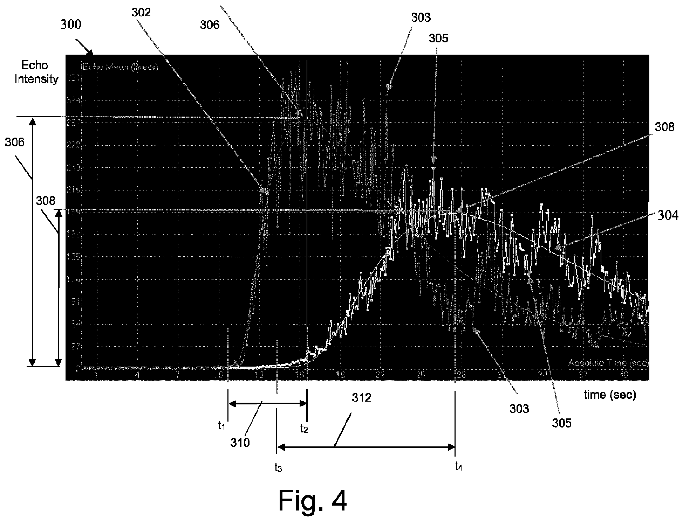

FIGURE 4 is a graph 300 showing first and second time-intensity curves 302 and 304 produced from echo signals acquired over time from contrast-perfused blood flow in the hepatic artery 12 and the portal vein 10, respectively. First contrast intensity information 303 (shown comprising connected points) was produced from harmonic signal returns acquired over time from the hepatic artery 12, and second contrast intensity information 305 was produced from harmonic signal returns acquired over time from the portal vein 10. The first and second contrast information values are processed by the ultrasound system, e.g., by averaging, curve fitting, *etc.*, using any suitable algorithm to respectively form the first and second time-intensity curves 302 and 304, respectively. Accordingly, the first time-intensity curve 302 corresponds with (*e.g.,* by fitting, averaging, *etc.*) the first contrast intensity information 303 and is, thus, an indication of the flow of contrast agent in the hepatic artery 12 over time. Similarly, the second time-intensity curve 304 corresponds with (*e.g.,* by fitting, averaging, *etc.*) the second contrast intensity information 305 and is, thus, an indication of the flow of contrast agent in the portal vein 10 over time.

Accordingly, the first time-intensity curve 302 correspond to the varying intensity of a first group of pixels of contrast flow in the first ROI drawn over the hepatic artery 12 in FIGURE 2 and is related to the flow of contrast agent in the hepatic artery over time. Similarly, the second time-intensity curve 304 corresponds to the varying intensity of a second group of pixels (i.e., those in the second ROI drawn over the portal vein 10 in FIGURE 3) and is related to the flow of contrast agent in the portal vein over time.

FIGURE 4 also illustrates a number of quantified parameters which may be obtained from the time-intensity curves (TICs). Indicated at 306 is the peak intensity of the TIC of the hepatic artery 12 and the time 310 from $t_1$ to $t_2$ is the time from the first arrival of contrast in the artery to the peak intensity, referred to as the wash-in time. The mean slope of the curve between times $t_1$ and $t_2$ may also be calculated if desired. Similarly, 308 indicates the peak intensity of the TIC of the portal vein 10 and the time interval 312 from $t_3$ to $t_4$ is the wash-in time from the first arrival of contrast in the vein to the peak intensity of contrast flow. Following the peak intensity points of both curves the curves decline as contrast agent washes out of the vessels. The durations and slopes of this portion of the curves may also be quantified if desired.

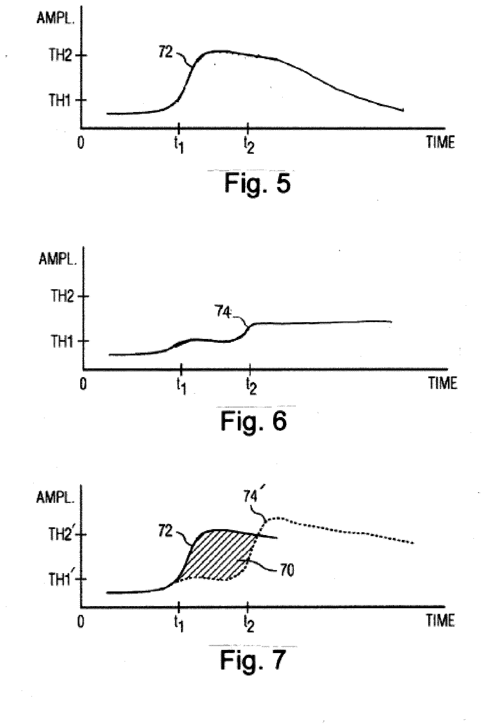

FIGURES 5 and 6 show examples of time-intensity curves for the hepatic artery 12 and portal vein 10, respectively. In FIGURE 5 the hepatic artery TIC 72 begins to rise from a level of threshold TH1 at a time $t_1$ to a peak threshold TH2 of peak contrast intensity. Thereafter the amount of contrast in the artery begins to decline, first slowly and then at a more rapid decline as the curve 72 illustrates. The portal vein TIC 74 in FIGURE 6 does not begin to rise until the later arrival of contrast in the portal vein at time $t_2$ and rises to a lesser peak intensity shortly thereafter, which is substantially sustained during the time of the curve 74 shown in the drawing.

The flow of contrast in the capillary structure of the liver parenchyma from these two sources of flow is a combination of both, as shown in FIGURE 7. The composite TIC in the parenchyma is curve 72 (from the hepatic arterial flow) and curve 74' where it exceeds the level of curve 72 after the arrival of the portal venous flow. Important in distinguishing between the two sources of flow is the shaded region 70 in FIGURE 7. This is a region demarcated by time interval $t_1$ - $t_2$, the time from the arrival of contrast from the hepatic artery to the later time of arrival of contrast from the portal vein. In FIGURE 4 this would be the time interval $t_1$-$t_3$. This time is a temporal window during which the blood flow in the liver is only arterial blood flow from the hepatic artery; after time $t_2$ in FIGURE 7, the flow in the liver is a combination of flow from both sources.

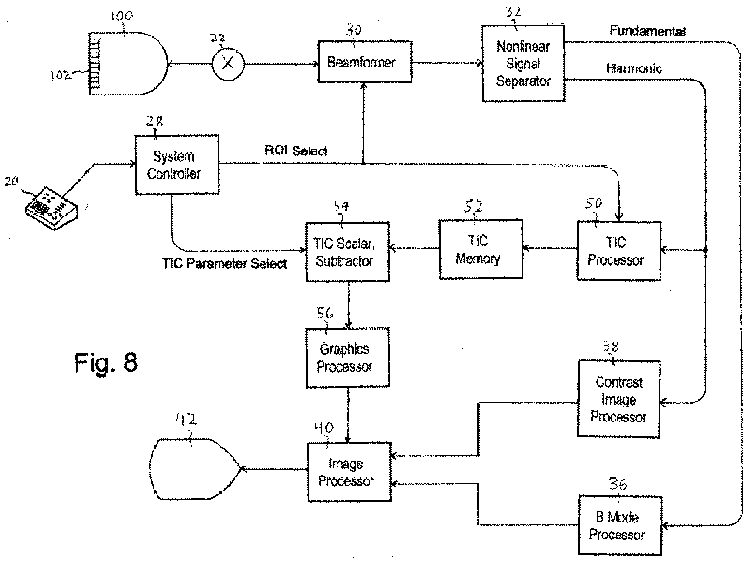

Referring now to FIGURE 8, an ultrasound system constructed in accordance with the present invention is shown in block diagram form. An ultrasonic probe 100 includes an array 102 of ultrasonic transducer elements that transmit and receive ultrasonic pulses. The array may be a one dimensional linear or curved array for two dimensional imaging, or may be a two dimensional matrix of transducer elements for electronic beam steering and focusing in two or three dimensions. The ultrasonic transducer elements in the array 102 transmit beams of ultrasonic energy by their timed actuation by a beamformer 30, and receive echoes returned in response to this transmission. A transmit/receive ("T/R") switch 22 is coupled to the ultrasonic transducers in the array 102 to selectively couple signals from the transducer elements to A/D converters in the beamformer 30 during the receive phase of operation. The times at which the transducer array is actuated to transmit signals may be synchronized to an internal system clock (not shown), or may be synchronized to a bodily function such as the heart cycle, for which a heart cycle waveform is conventionally provided by an ECG waveform sensor coupled to the ultrasound system. When the heartbeat is at the desired phase of its cycle as determined by the waveform provided by ECG sensor, the probe is commanded by a system controller to acquire an ultrasonic image.

Echoes from the transmitted ultrasonic energy are received by the transducers of the array 102, which generate echo signals that are coupled through the T/R switch 22 and digitized by the analog to digital converters when the system uses a digital beamformer. Analog beamformers may alternatively be used. The A/D converters sample the received echo signals at a sampling frequency controlled by a signal $f_s$ generated by a system controller 28. The desired sampling rate dictated by sampling theory is at least twice the highest frequency of the received passband, and might be on the order of 30-40 MHz. Sampling rates higher than the minimum requirement are also desirable. Control of the ultrasound system and of various control setting for imaging such as probe selection and ROI delineation is effected by user manipulation of the controls of a user control panel 20 which is coupled to and applies its control through the system controller 28.

The echo signal samples from the individual transducers of the array 14 are delayed and summed by the beamformer 30 to form coherent echo signals along scanline directions for an image. The digital coherent echo signals may then be filtered by a digital filter and undergo noise reduction as by spatial or frequency compounding. The digital filter can also shift the frequency band to a lower or baseband frequency range. The digital filter could be a filter of the type disclosed in U.S. Patent No. 5,833,613 (Averkiou et al.), for example. When phase information is needed as is the case for Doppler processing, quadrature (I and Q) demodulation may also be performed on the echo signals. In this implementation, the transmit frequency $f_o$ and the receiver frequency are individually controlled so that the beamformer 32 is free to receive a band of frequencies which is different from that of the transmitted band such as a harmonic frequency band around frequency $2f_o$.

The beamformed coherent echo signals are coupled to a nonlinear signal separator 32. The nonlinear signal separator 32 preferably separates harmonic frequency echoes returned from harmonic contrast agents by the pulse inversion technique, in which echo signals resulting from the transmission of multiple, differently phased (inverted) pulses to an image location are additively combined to cancel fundamental signal components and enhance harmonic components, thus producing echo signals in a harmonic band $2f_o$. The same echo signals are subtractively combined to produce echo signals in a fundamental frequency band $f_o$. A preferred pulse inversion technique is described in U.S. patent 6,186,950 (Averkiou et al.) and in U.S. patent 5,706,819 (Hwang et al.) for instance.

Harmonic echo signals from a contrast agent, such as microbubbles, are coupled to a contrast image processor 38. Contrast agents are often used to more clearly delineate blood vessels, or to perform perfusion studies of the microvasculature of tissue as described in US Pat. 6,692,438 (Skyba et al.) for example. In the implementation shown in FIGURE 8, echoes from a contrast agent are used to produce both a contrast image as shown in FIGURES 2 and 3, and time intensity curves from selected ROIs in the image field. The contrast agent processor produces a contrast image by amplitude (or envelope) detection of the harmonic frequency echoes from each point in the image field. One way to do this when the echoes are quadrature demodulated is to calculate the amplitude in the form of $(I^2+Q^2)^{1/2}$. These contrast intensity signals are mapped to the desired display format by scan conversion which converts samples from $R-\theta$ coordinates to Cartesian $(x,y)$ coordinates for display of a spatially defined image as shown in FIGURES 2 and 3.

The fundamental frequency echo signals are coupled to a B mode processor 36 which produces a standard B mode tissue image. The B mode processor performs in the same manner as the contrast image processor, but operates on fundamental frequency echoes. The echo signals are amplitude (envelope) detected and scan converted to produce a spatially delineated image of tissue such as the B mode images shown on the right side of FIGURES 2 and 3. The contrast and B mode images are coupled to an image processor 40 which does the image processing needed to display the images on an image display 42. This may include displaying both images at the same time, side-by-side, as shown in FIGURES 2 and 3. It may also comprise overlaying the contrast images over the B mode images so that the flow of blood containing contrast agent is shown in relation to the tissue structure of the vessels in which it is flowing.

In accordance with the present invention the harmonic echo signals are coupled to a TIC processor 50. The TIC processor processes echo signals from a contrast agent received over time from a region of interest (ROI) to produce a time-intensity curve such as those shown in FIGURES 4-7. As mentioned above, this processing involves acquiring echo signals over time from contrast agent washing into and out of the vessel(s) of a region of interest and fitting this echo data to a smooth curve. Since a contrast agent can be applied in a bolus injection, and can also be disrupted by relatively intense ultrasound which breaks or diffuses the microbubbles which are then allowed to reperfuse the vessels in the ROI, temporal characteristics of the arrival and departure of the contrast agent can be measured and used for diagnosis. A common measure is the time-intensity curve of the arrival and departure of the contrast agent as described in US Pat. 5,833,613 (Averkiou et al.) A time-intensity curve can be calculated for each point in an image of perfused tissue and one or more parameters of each curve for each image point can be displayed in grayscale shades or color-coding to form a parametric image of perfusion as described in US Pat. 6,692,438 (Skyba et al.) These parameters include the peak and the slope of the curves, each indicating a different characteristic of the tissue perfusion.

A time-intensity curve is generally computed by measuring the intensity of signals returned from the contrast agent as it flows into and out of blood vessels and the microvasculature of the tissue. These measurements of the rise and fall of the amount of contrast agent are then fitted to a curve such as that defined by the Gamma-variate curve model

$$A*(x-t_0)*exp(-\rho*(x-t_0))+C,$$

where $\mathbf{A}$ is the curve peak, $\mathbf{t_0}$ is the time of initiation of the increase of contrast agent, $\rho$ is the slope of the rise of the curve, and $\mathbf{x}$ is the instantaneous measurement of the amount of the contrast agent. These time and intensity representations provide an indication to a trained clinician of the manner in which the tissue is perfused.

In an implementation of the present invention a time-intensity curve is formed from the contrast wash-in and wash-out in an ROI located over the hepatic artery 12 as shown in FIGURE 2, and from the contrast wash-in and wash-out in an ROI located over the portal vein 10 as shown in FIGURE 3. This is done by selection of an ROI manually by the user by manipulating a control to move a cursor to the ROI in an image or by automated selection means, and providing the spatial location of the ROI by an "ROI Select" signal to the TIC processor. The ROI Select signal may also be provided to the beamformer to instruct the beamformer where to acquire the required echoes for time-intensity curve processing. The TIC from the hepatic artery will appear similar to that shown in FIGURE 5 (and as curve 302 in FIGURE 4), and the TIC from the portal vein will appear similar to that shown in FIGURE 6 (and as curve 304 in FIGURE 4). The resulting curves are stored as hepatic and portal reference curves in a TIC memory 52. Then a time-intensity curve is produced from the reperfusion of contrast in an ROI of the liver parenchyma. In accordance with the present invention, the flow into the parenchyma from the two sources of blood, the hepatic artery and the portal vein is then estimated. The reference curve acquired from the hepatic artery 12 is related to the TIC of the ROI which is a combination of the flow from both sources. Since the TIC from the hepatic artery is from contrast in pure blood flow, it will have a significantly greater intensity (amplitude) than the TIC from the liver ROI. The hepatic artery TIC (302,72) is therefore scaled in amplitude (and time, if needed) to match the amplitude of the liver TIC, principally using the portion of the hepatic artery TIC occurring during time window $t_1$-$t_2$ in FIGURES 5-7 or a part of such a portion when the flow in the liver is only from the hepatic artery. The thus-scaled hepatic artery TIC is then used as an estimate of the flow characteristic in the liver ROI resulting only from flow from the hepatic artery.

Theoritically, the arrival of contrast agent flow from the hepatic artery in different ROIs varies from each other depending on the location of the ROIs, e.g. earlier in ROIs nearer to the hepatic artery. Practically, due to the fast perfusion of the contrast agent flow, such differences can be neglected especially in cases when the differences are not significant. In some other embodiments, the hepatic artery TIC may be further scaled in time, by time shifting the curve for example. Particularly, time delay may be introduced because the arrival of contrast agent flow in liver parenchyma is theoritically later than the arrival of contrast agent flow in the hepatic artery. The amount of the time shift can be determined by comparing the hepatic artery TIC and the TIC in the ROI. For example, the arrival time of contrsat in each of the two curves can be determined as the time when the amplitude exceeds a predetermined threshold. In an embodiment, the predetermined threshold can be set as an absolute value. As the amplitude of the hepatic artery TIC is much greater than the amplitude of the TIC in the ROI, the predetermined threshold for determining the arrival of contrast in the hepatic artery is set as being different from that for determining the arrival of contrast in the ROI. In another embdoiment, the predetemrined threshold for determining the arrival of contrast can be set as a reletive value to the peak of the corresponding TIC.

Since the combined flow in the liver ROI is linearly related to the flow of blood from both sources, the scaled hepatic artery TIC is then subtracted from TIC from the liver ROI, which, for instance, is the combination of curves 72 and 74' in FIGURE 7. This subtraction will result in a curve similar to curve 74 of FIGURE 6 but at the lower amplitude (compared to the portal vein curve) of the tissue perfusion curve from the liver ROI. The resulting curve is then used as an estimate of the flow characteristic in the liver ROI resulting only from flow from the portal vein.

One or more characteristics of these estimated curves are coupled to a graphics processor 56 which displays them to the clinician for diagnostic purposes. A simple display is to display the data of the two estimated curves of arterial and venous perfusion for a selected ROI on the display screen, which will appear similar to the curves shown in FIGURES 5 and 6. A more complete two-dimensional map for the liver in the image field may also be produced. For instance, estimated arterial and venous curve data may be calculated for each point in the liver in an image, and a parameter of the curves shown qualitatively at each point in the liver as a parametric overlay over a B mode image. Using the Gamma-variate curve equation above, the peak of each curve A is selected and converted to a color value which varies with the magnitude of **A.** A B mode image may then be overlaid with a color map indicating the peak intensity **A** of arterial perfusion at each point in the liver, or overlaid with a color map indicating the peak intensity **A** of venous perfusion at each point in the liver image. Other parameters may also be calculated for each point in an image, color-coded, and displayed, such as a parametric map of wash-in time of arterial blood flow ($t_1$-$t_2$ in FIGURE 4), wash-in time of venous blood flow ($t_3$-$t_4$ in FIGURE 4), or the slope of the wash-in time ($\rho$) for either arterial or venous flow. Other parameters which may be displayed will be readily apparent to those skilled in the art.

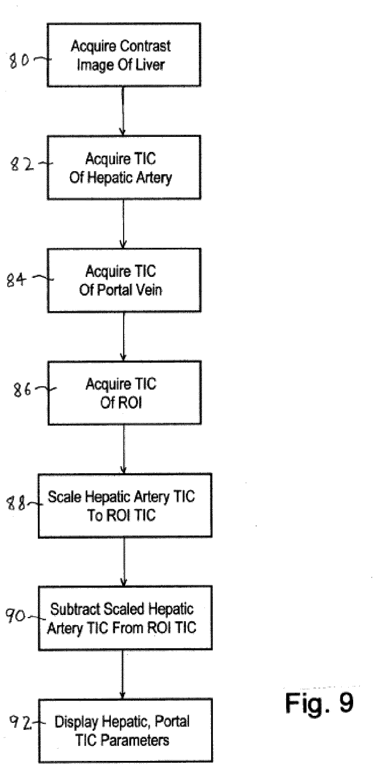

The steps of an exemplary method in accordance with the present invention is shown in FIGURE 9. In step 80 contrast images of the liver are acquired from a source of contrast-infused liver images, such as those shown in FIGURES 2 and 3. These may be live (real time) images or an image loop previously acquired and retrieved from storage in memory. In step 82 a time-intensity curve of flow in the hepatic artery is acquired. This may be done by placing an ROI over the hepatic artery 12 in the images as shown in FIGURE 2, acquiring contrast echo data from the hepatic flow during wash-in and wash-out, and processing the data in the TIC processor 50 to produce an arterial reference TIC. In step 84 a time-intensity curve of flow in the portal vein is acquired. This may be done by placing an ROI over the portal vein 10 in the images as shown in FIGURE 3, acquiring contrast echo data from the venous flow during wash-in and wash-out, and processing the data in the TIC processor 50 to produce a venous reference TIC. In step 86 a time-intensity is acquired from the flow in a liver ROI. As mentioned above, only one liver ROI may be used, or time-intensity curves acquired over a portion or all of the liver parenchyma. In step 88 the larger amplitude arterial TIC is scaled to the size and duration of the liver ROI curve, using the period of initial wash-in when flow in the liver is due only to hepatic arterial flow. The scaled arterial TIC data is used as an estimated time-intensity curve of flow in the ROI due to blood from only the hepatic artery. In step 90 the scaled arterial TIC data is subtracted from the liver ROI curve to produce time-intensity curve data which is an estimate of the blood flow in the liver ROI due to only venous flow. In step 92 one or more parameters of the arterial and venous flow curves are displayed to the user, such as displaying the estimated arterial and venous curves of a single ROI in the liver, or a parametric map of time-intensity curve parameters for some or all of the liver on the display.

It should be noted that the ultrasound system which acquires contrast data from flow in the hepatic artery, portal vein, and liver and produces estimates of flow in the liver due to the individual sources of blood flow as shown in FIGURE 8 and in the method of FIGURE 9, and in particular the component structure of the ultrasound system of FIGURE 8, may be implemented in hardware, software or a combination thereof. The various embodiments and/or components of an ultrasound system, for example, the modules, or components and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system. The computer or processor may also include a memory. The memory devices such as the A-line memory 24 may include Random Access Memory (RAM) and Read Only Memory (ROM). The computer or processor further may include a storage device, which may be a hard disk drive or a removable storage drive such as a floppy disk drive, optical disk drive, solid-state thumb drive, and the like. The storage device may also be other similar means for loading computer programs or other instructions into the computer or processor.

As used herein, the term "computer" or "module" or "processor" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including the acquisition of contrast data and the calculation of time-intensity curves and parameters described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules, a program module within a larger program or a portion of a program module. For example, the equations calculated by the time-intensity data processors of FIGURE 8 may be executed by software modules calculating the equations. The software also may include modular programming in the form of object-oriented programming. The processing of input data by the processing machine may be in response to operator commands, or in response to results of previous processing, or in response to a request made by another processing machine. In the imaging and time-intensity calculation portions of FIGURE 8, for instance, software instructions are conventionally employed to calculate the equations given above.

**Claims**

1.  A system for characterizing liver perfusion of contrast agent flow on basis of ultrasound data acquired from a liver in the presence of contrast agent flow, comprising:

    a first source of time-intensity curves of contrast agent flow in a hepatic artery, and in at least one region of interest, i.e. ROI, in liver parenchyma of the liver, the time-intensity curves being produced from the ultrasound data;

    being **characterized by**:
    a time-intensity curve scalar and subtracter, configured to produce, for an ROI of the at least one ROI, time-intensity curve data representative of arterial flow in the ROI by scaling the time-intensity curve of contrast agent flow in the hepatic artery to the time-intensity curve of contrast agent flow in the ROI, and to produce time-intensity curve data representative of venous flow in the ROI by subtracting the time-intensity curve data representative of arterial flow in the ROI from the time-intensity curve of contrast agent flow in the ROI.

2.  The system of Claim 1, wherein the first source comprises:

    a second source of ultrasound liver image data acquired in the presence of contrast agent flow;
    a nonlinear signal separator, coupled to receive the ultrasound liver image data, and configured to produce harmonic echo signal data from contrast agent flow; and
    a time-intensity curve processor, responsive to the harmonic echo signal data, which is configured to produce the time-intensity curves of contrast agent flow in the hepatic artery, and in the at least one ROI.

3.  The system of Claim 1, wherein the time-intensity curve scalar and subtractor is further configured to scale the whole time-intensity curve of contrast agent flow in the hepatic artery in amplitude such that the amplitude of the time-intensity curve of contrast agent flow in the hepatic artery matches the amplitude of the time-intensity in the ROI in a region or a part of the region, which region is demarcated by the time interval from the arrival of contrast from the hepatic artery to the later time of arrival of contrast from a portal vein of the liver.

4.  The system of Claim 3, wherein
    the first source is further configured to provide a time-intensity curve of contrast agent flow in the portal vein of the liver; and
    the time-intensity curve scalar and subtractor is further configured to determine the arrival of contrast from the hepatic artery on basis of the time-intensity curve of contrast agent flow in the hepatic artery, and to determine the later time of arrival of contrast from the portal vein on basis of the time-intensity curve of contrast agent flow in the portal vein of the liver.

5.  The system of Claim 3, wherein the time-intensity curve scalar and subtractor is further configured to scale the time-intensity curve of contrast agent flow in the hepatic artery in time.

**6.** The system of Claim 1, further comprising: an output unit for outputting one or more of the time-intensity curve data representative of arterial flow in the ROI and the time-intensity curve data representative of venous flow in the ROI.

**7.** The system of Claim 6, wherein the output unit is further configured to ouput a time-intensity curve representative of arterial flow in the ROI and a time-intensity curve representative of venous flow in the ROI.

**8.** The ultrasonic diagnostic imaging system of Claim 6, wherein the output unit is further configured to output a parametric map of arterial flow or venous flow in a liver.

**9.** A method for characterizing liver perfusion of contrast agent flow on basis of ultrasound liver image data acquired from a liver in the presence of contrast agent flow, comprising:

acquiring a time-intensity curve of contrast agent flow in a hepatic artery location, and a time-intensity curve of contrast agent flow in each of at least one region of interest, i.e. ROI, in the liver parenchyma of the liver, the time-intensity curves being produced from the ultrasound liver image data;
being **characterized by**, for a ROI of the at least one ROI:

producing time-intensity curve data representative of arterial flow in the ROI by scaling the time-intensity curve of contrast agent flow in the hepatic artery location to match the time-intensity curve of contrast agent flow in the ROI; and
producing time-intensity curve data representative of venous flow in the ROI by subtracting the time-intensity curve data representative of arterial flow in the ROI from the time-intensity curve of contrast agent flow in the ROI.

**10.** The method of Claim 9, further comprising selecting a ROI in an image of the liver.

**11.** The method of Claim 9, further comprising:

acquiring contrast agent flow images of the liver;
producing harmonic echo signal data from the contrast agent flow on basis of the contrast agent flow images; and
producing the time-intensity curve of contrast agent flow in the hepatic artery location, and the time-intensity curve of contrast agent flow in each ROI of the at least one ROI from the harmonic echo signal data.

**12.** The method of Claim 9, wherein scaling further comprises matching an initial rise of the time-intensity curve of contrast agent flow in the hepatic artery location to an initial rise of the time-intensity curve of contrast agent flow in the ROI.

**13.** The method of Claim 12, wherein
the initial rise is demarcated by a time interval or a part of the time interval from the arrival of contrast from the hepatic artery to the later time of arrival of contrast from a portal vein of the liver;
the method further comprises acquiring a time-intensity curve of contrast agent flow in the portal vein location, and determining the later time of arrival of contrast from the portal vein on basis of the time-intensity curve of contrast agent flow in the portal vein location.

**14.** The method of Claim 9 wherein further comprises producing a parametric map of one or more of the time-intensity curve data representative of arterial flow in the liver and the time-intensity curve data representative of venous flow in the liver.

**15.** A computer program product comprising instructions which, when the computer program product is executed by a computer, cause the computer to carry out the method of any of Claims 9-14.

**Patentansprüche**

**1.** Kontrastmittelfluss anhand der Ultraschalldaten einer Leber, durch die Kontrastmittel fließt, das Folgendes umfasst:

eine erste Quelle für Zeit-Intensität-Kurven für den Kontrastmittelfluss in einer Leberarterie und in mindestens einem Untersuchungsbereich, d. h. UB, im Leberparenchym der Leber, wobei die Zeit-Intensität-Kurven auf Ultraschalldaten beruhen;
die sich durch Folgendes auszeichnet:
einen Skalar und einen Subtraktor der Zeit-Intensität-Kurve, mit denen für den einen UB von mindestens einem UB die Zeit-Intensität-Kurvendaten erstellt werden, die der arteriellen Durchblutung im UB entsprechen, indem die Zeit-Intensität-Kurve des Kontrastmittelflusses in der Leberarterie auf die Zeit-Intensität-Kurve des Kontrastmittelflusses im UB skaliert wird. Zudem werden die Zeit-Intensität-Kurvendaten erstellt, die der venösen Durchblutung im UB entsprechen, indem die Zeit-Intensität-Kurvendaten, die der arteriellen Durchblutung im UB entsprechen, von der Zeit-Intensität-Kurve des Kontrastmittelflusses im UB abgezogen werden.

2. Das System gemäß Anspruch 1, wobei die erste Quelle Folgendes umfasst:

eine zweite Quelle für Ultraschallbilddaten der Leber, die bei Kontrastmittelfluss erfasst wurden;

ein angeschlossener nicht linearer Signaltrenner, der die Ultraschallbilddaten der Leber empfängt und harmonische Echosignaldaten des Kontrastmittelflusses erstellt; und

ein Zeit-Intensität-Kurven-Prozessor, der auf die harmonischen Echosignaldaten reagiert und die Zeit-Intensität-Kurven des Kontrastmittelflusses in der Leberarterie und in mindestens einem UB erstellt.

3. Das System gemäß Anspruch 1, wobei der Skalar und Subtraktor der Zeit-Intensität-Kurve zudem die gesamte Zeit-Intensität-Kurve des Kontrastmittelflusses in der Leberarterie in einem solchen Umfang skalieren, dass die Amplitude der Zeit-Intensität-Kurve des Kontrastmittelflusses der Leberarterie der Amplitude der Zeit-Intensität des UB in einem Bereich oder Teilbereich entsprechen, der vom Zeitintervall vom Beginn des Kontrasts in der Leberarterie bis zum Beginn des Kontrasts in einer Pfortader der Leber reicht.

4. Das System gemäß Anspruch 3, wobei:

die erste Quelle zudem eine Zeit-Intensität-Kurve des Kontrastmittelflusses in der Pfortader der Leber bereitstellt; und

der Skalar und Subtraktor der Zeit-Intensität-Kurve zudem den Beginn des Kontrasts in der Leberarterie auf Grundlage der Zeit-Intensität-Kurve des Kontrastmittelflusses in der Leberarterie sowie den Beginn des Kontrasts in der Pfortader auf Grundlage der Zeit-Intensität-Kurve des Kontrastmittelflusses in der Pfortader der Leber ermitteln.

5. Das System gemäß Anspruch 3, wobei der Skalar und Subtraktor der Zeit-Intensität-Kurve zudem die Zeit-Intensität-Kurve des Kontrastmittelflusses in der Leberarterie zeitlich skalieren.

6. Das System gemäß Anspruch 1, wobei dieses zudem Folgendes umfasst:
eine Ausgabeeinheit für das Ausgeben mindestens einer der Zeit-Intensität-Kurvendaten, die jeweils der arteriellen im UB und venösen Durchblutung im UB entsprechen.

7. Das System gemäß Anspruch 6, wobei die Ausgabeeinheit zudem jeweils eine Zeit-Intensität-Kurve für die arterielle im UB und venöse Durchblutung im UB entsprechen.

8. Das diagnostische Ultraschall-Bildgebungssystem gemäß Anspruch 6, wobei die Ausgabeeinheit zudem eine parametrische Karte der arteriellen oder venösen Durchblutung der Leber ausgibt.

9. Eine Methode zum Beschreiben der Leberdurchblutung mithilfe von Kontrastmittel anhand der Ultraschallbilddaten einer Leber, durch die Kontrastmittel fließt, die Folgendes umfasst:

Abrufen einer Zeit-Intensität-Kurve für den Kontrastmittelfluss in einer Leberarterie sowie in den einzelnen von mindestens einem Untersuchungsbereich, d. h. UB, im Leberparenchym der Leber, wobei die Zeit-Intensität-Kurven auf den Ultraschall-Bilddaten der Leber beruhen;
die sich für den UB der mindestens einen UB durch Folgendes auszeichnet:

Erstellen der Zeit-Intensität-Kurvendaten der arteriellen Durchblutung im UB, indem die Zeit-Intensität-Kurve des Kontrastmittelflusses in der Leberarterie so skaliert wird, dass sie mit der Zeit-Intensität-Kurve des Kontrastmittelflusses im UB übereinstimmt; und
Erstellen der Zeit-Intensität-Kurvendaten der venösen Durchblutung im UB, indem die Zeit-Intensität-Kurvendaten der arteriellen Durchblutung im UB von der Zeit-Intensität-Kurve des Kontrastmittelflusses im UB abgezogen wird.

10. Die Methode gemäß Anspruch 9, wobei diese zudem das Auswählen eines UB auf einem Bild der Leber umfasst.

11. Die Methode gemäß Anspruch 9, wobei diese zudem Folgendes umfasst:

Abrufen von Kontrastmittelfluss-Bildern der Leber;
Erstellen von harmonischen Echosignaldaten für den Kontrastmittelfluss anhand der Kontrastmittelfluss-Bilder; und
Erstellen der Zeit-Intensität-Kurve für den Kontrastmittelfluss in einer Leberarterie sowie in den einzelnen UB von mindestens einem UB anhand der harmonischen Echosignaldaten.

12. Die Methode gemäß Anspruch 9, wobei beim Skalieren zudem der erste Anstieg der Zeit-Intensität-Kurve des Kontrastmittelflusses in der Leberarterie mit dem ersten Anstieg der Zeit-Intensität-Kurve des Kontrastmittelflusses im UB abgeglichen wird.

13. Die Methode gemäß Anspruch 12, wobei der erste Anstieg über ein Zeitintervall oder einen Teil eines Zeitintervalls vom Beginn des Kontrasts in der Leberarterie bis zum Beginn des Kontrasts in einer Pfortader der Leber reicht; diese zudem das Abrufen einer Zeit-Intensität-Kurve des Kontrastmittelflusses in der Pfortader sowie das Bestimmen des Beginns des Kontrasts in der Pfortader auf Grundlage der Zeit-Intensität-Kurve des Kontrastmittelflusses in der Pfortader umfasst.

14. Die Methode gemäß Anspruch 9, wobei diese zudem das Erstellen einer parametrischen Karte mindestens einer der Zeit-Intensität-Kurvendaten umfasst, die jeweils der arteriellen und venösen Durchblutung in der Leber entsprechen.

15. Ein Computerprogramm, dass Anweisungen umfasst, die den Computer beim Ausführen des Computerprogramms dazu veranlassen, die Methode gemäß der Ansprüche 9 bis 14 auszuführen.

**Revendications**

1. Système de caractérisation de la perfusion hépatique d'un écoulement d'agent de contraste en fonction des données ultrasonores acquises à partir d'un foie en présence d'un écoulement d'agent de contraste, comprenant :

   une première source de courbes d'intensité temporelle de l'écoulement d'agent de contraste dans une artère hépatique et dans au moins une zone d'intérêt, ROI, dans le parenchyme hépatique du foie, les courbes d'intensité temporelle étant générées à partir des données ultrasonores ;
   **caractérisé par** :
   un scalaire et soustracteur de courbe d'intensité temporelle, configuré pour générer, pour une ROI de l'au moins une ROI, des données de courbe d'intensité temporelle représentatives de l'écoulement artériel dans la ROI par mise à l'échelle de la courbe d'intensité temporelle de l'écoulement d'agent de contraste dans l'artère hépatique par rapport à la courbe d'intensité temporelle de l'écoulement d'agent de contraste dans la ROI, et pour générer des données de courbe d'intensité temporelle représentatives de l'écoulement veineux dans la ROI par soustraction des données de courbe d'intensité temporelle représentatives de l'écoulement artériel dans la ROI de la courbe d'intensité temporelle de l'écoulement d'agent de contraste dans la ROI.

2. Système selon la revendication 1, dans lequel la première source comprend :

   une seconde source d'images par ultrasons du foie acquises en présence d'un écoulement d'agent de contraste ;
   un séparateur de signal non linéaire, couplé pour recevoir les données d'image du foie par ultrasons, et configuré pour générer des données de signal d'écho harmonique à partir de l'écoulement d'agent de contraste ; et
   un processeur de courbe d'intensité temporelle, sensible aux données de signal d'écho harmonique, lequel est configuré pour générer les courbes d'intensité temporelle de l'écoulement d'agent de contraste dans l'artère hépatique et dans au moins une ROI.

3. Système selon la revendication 1, dans lequel le scalaire et soustracteur de courbe d'intensité temporelle est en outre configuré pour mettre à l'échelle toute la courbe d'intensité temporelle de l'écoulement d'agent de contraste dans l'artère hépatique en amplitude de telle sorte que l'amplitude de la courbe d'intensité temporelle de l'écoulement d'agent de contraste dans l'artère hépatique correspond à l'amplitude de l'intensité de temps dans la ROI dans une zone ou une partie de la zone, ladite zone étant délimitée par un intervalle de temps entre l'arrivée de contraste de l'artère hépatique et l'heure ultérieure d'arrivée de contraste d'une veine porte du foie.

4. Système selon la revendication 3, dans lequel la première source est en outre configurée pour fournir une courbe d'intensité temporelle de l'écoulement d'agent de contraste dans la veine porte du foie ; et le scalaire et soustracteur de courbe d'intensité temporelle est en outre configuré pour déterminer l'arrivée de contraste de l'artère hépatique en fonction de la courbe d'intensité temporelle de l'écoulement d'agent de contraste dans l'artère hépatique, et pour déterminer l'heure d'arrivée de contraste de la veine porte en fonction de la courbe d'intensité temporelle de l'écoulement d'agent de contraste dans la veine porte du foie.

5. Système selon la revendication 3, dans lequel le scalaire et soustracteur de courbe d'intensité temporelle est en outre configuré pour mettre à l'échelle la courbe d'intensité temporelle de l'écoulement d'agent de contraste dans l'artère hépatique dans le temps.

6. Système selon la revendication 1, comprenant en outre :

   une unité de sortie pour sortir des données de courbe d'intensité temporelle représentatives de l'écoulement artériel dans la ROI et/ou des données de courbe d'intensité temporelle représentatives de l'écoulement veineux dans la ROI.

**7.** Système selon la revendication 6, dans lequel l'unité de sortie est en outre configurée pour générer une courbe d'intensité temporelle représentative de l'écoulement artériel dans la ROI et une courbe d'intensité temporelle représentative de l'écoulement veineux dans la ROI.

**8.** Système d'imagerie diagnostique par ultrasons selon la revendication 6, dans lequel l'unité de sortie est en outre configurée pour générer une carte paramétrique de l'écoulement artériel ou de l'écoulement veineux dans un foie.

**9.** Procédé de caractérisation de la perfusion hépatique d'un écoulement d'agent de contraste en fonction des données d'image hépatique par ultrasons acquises à partir d'un foie en présence d'un écoulement d'agent de contraste, comprenant :

l'acquisition d'une courbe d'intensité temporelle de l'écoulement d'agent de contraste dans un emplacement de l'artère hépatique, et d'une courbe d'intensité temporelle de l'écoulement d'agent de contraste dans chaque zone d'au moins une zone d'intérêt, ROI, dans le parenchyme hépatique du foie, les courbes d'intensité temporelle étant générées à partir des données d'image par ultrasons du foie ;

**caractérisé par**, pour une ROI de l'au moins une ROI :

la génération des données de courbe d'intensité temporelle représentatives de l'écoulement artériel dans la ROI par mise à l'échelle de la courbe d'intensité temporelle de l'écoulement d'agent de contraste dans l'emplacement de l'artère hépatique pour correspondre à la courbe d'intensité temporelle de l'écoulement d'agent de contraste dans la ROI ; et

la génération des données de courbe d'intensité temporelle représentatives de l'écoulement veineux dans la ROI par soustraction des données de courbe d'intensité temporelle représentatives de l'écoulement artériel dans la ROI de la courbe d'intensité temporelle de l'écoulement d'agent de contraste dans la ROI.

**10.** Procédé selon la revendication 9, comprenant en outre la sélection d'une ROI dans une image du foie.

**11.** Procédé selon la revendication 9, comprenant en outre :

l'acquisition des images de l'écoulement d'agent de contraste du foie ;

la génération des données de signal d'écho harmonique à partir de l'écoulement d'agent de contraste en fonction des images de l'écoulement d'agent de contraste ; et

la génération de la courbe d'intensité temporelle de l'écoulement d'agent de contraste à l'emplacement de l'artère hépatique, et de la courbe d'intensité temporelle de l'écoulement d'agent de contraste dans chaque ROI de l'au moins une ROI à partir des données de signal d'écho harmonique.

**12.** Procédé selon la revendication 9, dans lequel la mise à l'échelle comprend en outre la correspondance d'une élévation initiale de la courbe d'intensité temporelle de l'écoulement d'agent de contraste à l'emplacement de l'artère hépatique avec une élévation initiale de la courbe d'intensité temporelle de l'écoulement d'agent de contraste dans la ROI.

**13.** Procédé selon la revendication 12, dans lequel l'élévation initiale est délimitée par un intervalle de temps ou une partie de l'intervalle de temps depuis l'arrivée de contraste de l'artère hépatique jusqu'à l'heure ultérieure d'arrivée de contraste d'une veine porte du foie ;

ledit procédé comprend en outre l'acquisition d'une courbe d'intensité temporelle de l'écoulement d'agent de contraste à l'emplacement de la veine porte et la détermination de l'heure ultérieure d'arrivée du contraste à partir de la veine porte en fonction de la courbe d'intensité temporelle de l'écoulement d'agent de contraste à l'emplacement de la veine porte.

**14.** Procédé selon la revendication 9, dans lequel ledit procédé comprend en outre la génération d'une carte paramétrique des données de courbe d'intensité temporelle représentatives de l'écoulement artériel dans le foie et des données de courbe d'intensité temporelle représentatives de l'écoulement veineux dans le foie.

**15.** Produit de programme informatique comprenant des instructions, lesquelles, lorsque le produit de programme informatique est exécuté par un ordinateur, amènent l'ordinateur à mettre en œuvre le procédé selon l'une quelconque des revendications 9 à 14.

Fig. 1

Fig. 2

Fig. 3

Fig. 4

**Fig. 5**

**Fig. 6**

**Fig. 7**

Fig. 8

80 — Acquire Contrast
Image Of Liver

82 — Acquire TIC
Of Hepatic Artery

84 — Acquire TIC
Of Portal Vein

86 — Acquire TIC
Of ROI

88 — Scale Hepatic Artery TIC
To ROI TIC

90 — Subtract Scaled Hepatic
Artery TIC From ROI TIC

92 — Display Hepatic, Portal
TIC Parameters

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011041244 A1
- US 5833613 A, Averkiou
- US 6186950 B, Averkiou
- US 5706819 A, Hwang
- US 6692438 B, Skyba

**Non-patent literature cited in the description**

- **M. AVERKIOU et al.** Ultrasound Contrast Imaging Research. *Ultrasound Quarterly,* 2003, vol. 19 (1), 27
- **G. J. LUECK et al.** Hepatic Perfusion Imaging Using Factor Analysis of Contrast Enhanced Ultrasound. *IEEE TRANSACTIONS ON MEDICAL IMAGING,* 2008, vol. 27 (10), 1449